Europäisches Patentamt

(19) European Patent Office

Office européen des brevets

(11) Publication number: **0 356 192 A2**

(12) **EUROPEAN PATENT APPLICATION**

(21) Application number: **89308477.2**

(22) Date of filing: **22.08.89**

(51) Int. Cl.⁵: **A 23 K 1/17**
**A 61 K 31/33, A 61 K 31/71**

(30) Priority: **24.08.88 US 235844   24.08.88 US 236067**

(43) Date of publication of application:
**28.02.90  Bulletin  90/09**

(84) Designated Contracting States:
**AT BE CH DE ES FR GB GR IT LI LU NL SE**

(71) Applicant: **SMITHKLINE BEECHAM CORPORATION**
**One Franklin Plaza**
**Philadelphia Pennsylvania 19103  (US)**

(72) Inventor: **Hedde, Richard Dwane**
**799 Locust Grove Road**
**West Chester, PA 19382  (US)**

**Quach, Roseanna H. N.**
**112 West Hill Top Road**
**West Chester, PA 19382  (US)**

(74) Representative: **Giddings, Peter John, Dr. et al**
**Smith Kline & French Laboratories Ltd. Corporate**
**Patents Mundells**
**Welwyn Garden City Hertfordshire AL7 1EY  (GB)**

Claims for the following Contracting States: ES + GR.

(54) **Prevention & treatment of liver abscesses in animals.**

㊳ This invention relates to pharmaceutical compositions for use in the prevention and treatment of liver abscesses in animals by combining a first component which is a member of the Virginiamycin family of antibiotics with a second component which is a member of the ionophore family of antibiotics and to the use of a member of the Virginiamycin family of antibiotics in the manufacture of a medicament for preventing and treating liver abscesses in animals.

EP 0 356 192 A2

Description

# PREVENTION AND TREATMENT OF LIVER ABSCESSES IN ANIMALS

## Field of the Invention

This invention relates to the use of an antibiotic which is a member of the Virginiamycin family of antibiotics in the manufacture of a medicament for preventing and treating liver abscess in animals. This invention further relates to pharmaceutical compositions for use in the prevention and treatment of liver abscesses in animals by combining a first component which is a member of the Virginiamycin family of antibiotics with a second component which is a member of the ionophore family of antibiotics.

## Background of the Invention

The Virginiamycin family of antibiotics have generally been employed in the treatment of bacterial infections wherein the causative organism is a gram-positive bacteria and as a growth promotant in swine, poultry and beef cattle. Microbiological Reviews, 43, pp. 145-198 (1979); Maehr et al., Journal of Antibiotics, 361 (1979) and Russel, European Microbiological Societies, 23, pp. 289-291 (1984). However, the art does not disclose or suggest that the Virginiamycin family of antibiotics would be useful in treating liver abscesses in animals wherein the gram-negative bacteria Fusobacterium necrophorum appears to be the predominant causative organism.

The ionophore family of antibiotics have generally been employed as a growth promotant in feedlot cattle. Olentin, Feed management 33, pp. 14-24:, Brown et al., Journal of Animal Science, 37, pp. 1085-1091 (1973). However, the ionophore family of antibiotics have not provided a satisfactory means for controlling liver abscesses in cattle or sheep.

The major causative agent for liver abscesses in animals is Fusobacterium necrophorum, also referred to as Fusiformis necrophorus, Bacillus necrophorus or Sphaerophorus necrophorus. Fusobacterium necrophorum has been divided into three biotypes, namely A, B and C, of which only biotyloes A and B have been implicated in the formation of liver abscesses (Scanlan et al., Cornell Vet. 1983, 73, 288-297). This organism is widespread in nature and apparently proliferates rapidly at the sites of wounds in animals or on the skin or mucous membrane of animals. The microorganism can enter the bloodstream through cuts and lesions in the hoof-area of animals. The organism may be found in the digestive tract of healthy animals, in manure and in soil. The bacteria can also enter the bloodstream through a lesion that may form in the rumen as a result of the ingestion of a high concentrate diet. The bacteria which enters the bloodstream will be filtered out by the liver and thus eventually colonize and grow in the liver. The animal which has been most affected by abscessed livers are beef cattle and dairy cattle. Hale, "Liver Abscesses and Founder," Animal Health Nutrition, September (1985).

Liver abscesses were originally not considered to be a major problem in animals until a high concentrate diet became a common method of feeding such animals. The incidence of liver abscesses in feed lot cattle, for instance, ranges from about 18 to about 20 percent in such cattle. Nearly 55 percent of the total condemned livers in cattle are due to the formation of liver abscesses in such cattle. Hall, supra.

Antibiotics and chemotherapeutic agents that have been utilized with some success in the treatment of liver abscesses include: Tetracycline antibiotics, Sulphanomides, Pyrazinines, Monensin and Tylosin. Poter et al., Journal of Animal Science, 61, pp. 1058-1065 (1985); Brown et al., Journal of Animal Science, 37, pp. 1085-1091 (1973); and Adam, U.S. Patent No. 4,061,751 (December 6, 1977). Such treatments have not, however, provided entirely satisfactory control of liver abscesses in animals.

Accordingly, it has now been discovered that antibiotics from the Virginiamycin family of antibiotics either alone or in combination with a member of the ionophore family of antibiotics are effective in the treatment and prevention of liver abscesses in beef and dairy cattle, wherein the gram -negative bacterium, Fusobacterium necrophorum appears to be the predominant causative organism.

## Summary of the Invention

This invention relates to the use of an antibiotic in the manufacture of a medicament from the Virginiamycin family of antibiotics for treating and preventing liver abscesses in animals. A representative antibiotic from the Virginiamycin family of antibiotics is Virginiamycin factor M. This invention further relates to the use of an antibiotic from the Virginiamycin family of antibiotics in the manufacture of a medicament for treating and preventing liver abscesses in beef cattle and dairy cattle, wherein the gram-negative bacteria Fusobacterium necrophorum appears to be the predominant causative organism.

This invention also relates to pharmaceutical compositions for use in treating and preventing liver abscesses in animals by combining a first component which is a member of the Virginiamycin family of antibiotics with a second component which is a member of the ionophore family of antibiotics wherein the gram-negative bacteria Fusobacterium necrophorum appears to be the predominant causative organism. The combination of a first component which is a member of the Virginiamycin family of antibiotics with a second component which is a member of the ionophore family of antibiotics will provide a synergistic effect in the treatment of liver abscesses in animals in need thereof.

## Detailed Description of the Invention

According to this invention, in order to effect improvement in and to prevent the formation of liver abscesses in animals, an effective amount of an antibiotic from the Virginiamycin family of antibiotics is administered to the animal. In addition, it has been discovered that the combination of a first component which is a member of the Virginamycin family of antibiotics with a second component which is a member of the ionophore family of antibiotics will provide a synergistic effect in the treatment and prevention of liver abscesses in animals wherein Fusobacterium necrophorum appears to be the predominant causative agent.

An "effective amount" as used herein will be that amount which when delivered in vivo to animals causes an observable improvement to a liver abscess in such animals or prevents the formation of a liver abscess in such animals.

Each of the antibiotics from the Virginiamycin family of antibiotics which can be employed in this invention are known in the prior art. Many are commercially available and are employed in the treatment of gram-positive bacterial infections in animals as well as in the stimulation of growth in domestic animals such as poultry, swine, sheep and cattle.

Each antibiotic from the Virginiamycin family of antibiotics may be assigned to either one of the two basic primary structures, A and B. Although the two structures are structurally different, they each contain a macrocyclic lactone peptolide chemical structure. The A primary structures are polyunsaturated cyclic peptolides which can be considered as highly modified depsipeptides having a molecular weight of about 500. The members of this group were established by X-ray crystallography and mass spectrometry and by identification of the hydrolysis products. The B structures are generally characterized as cyclic hexadepsipeptides having a molecular weight of about 800. The B structures were largely established by chemical identification of the hydrolysis products.

The following Table 1 is representative of various A and B groups from the Virginiamycin family of antibiotics which may be used in this invention.

Table I

Antibiotics from the Virginiamycin Family of Antibiotics

| Complex antibiotic | Type A components | Type B components |
|---|---|---|
| Madumycin (A2315A) | Madumycin II | Madumycin I |
| Mikamycin | Mikamycin A | Mikamycin B |
| Ostreogrycin (E129) | Ostreogrycins A,C,D,G,Q | Ostreogrycins B (B1,B2,B3) |
| Patricin | | Patricins A and B |
| Plauracin | Plauracin II | Plauracin I |
| Pristinamycin (Pyostacin) | Pristinamycins II (A and B) | Pristinamycins I (A, B and C) |
| Streptogramin | Streptogramin A | Streptogramin B |
| Synergistins (PA114) | Synergistin A | Synergistins B (1 and 3) |
| Vernamycin | Vernamycin A | Vernamycins B (a, , , ) |
| | Griseoviridin | Viridogrisein (Etamycin) (Doricin) (C) |
| Virginiamycin (Staphylomycin) | Virginiamycins M (1 and 2) | Virginiamycins S (1, 2, 3, and 4) |

The complete list of antibiotics from the Virginiamycin family of antibiotics which may be used in this invention, as well as representative structures, is extensively reviewed and set forth by Cocito in Microbiological Reviews, June, pp. 145-198 (1979). This reference is incorporated herein by reference, and is illustrative of the members of the Virginiamycin family of antibiotics which can be employed in this invention.

It is further contemplated that many of the antibiotics from the Virginiamycin family of antibiotics may contain more than one factor. For example, Virginiamycin may contain components referred to as Virginiamycin M1 and M2 (collectively known as Factor M) as well as Virginiamycins S1, S2, S3 and S4 (Collectively known as Factor S). Similarly, the Virginiamycin-type antibiotic known as "A-2315" may contain factors A, B and C (see U.S. Patents 3,923,980 and U.S. 4,336,250). Accordingly, the various factors of each of the antibiotics from the Virginiamycin family of antibiotics are contemplated for use in this invention. Thus, it is contemplated that any reference to an antibiotic contained herein will include within such term all the various factors which can be employed individually or in combination with one another, or as a complex of all such factors.

The preferred antibiotics from the Virginiamycin family of antibiotics include Madumycin, Mikamycin, Ostreogrycin, Patricin, Plauracin, Pristinamycin, Streptogramin, Synergistins, Vernamycin or Virginiamycin, or factors thereof.

Especially preferred is the Factor M component of Virginiamycin.

Each of the antibiotics from the ionophore family of antibiotics which can be employed in this invention are known in the prior art. A number of these compounds are in commercial use and were originally used as anticoccidial feed additives for poultry. Each of the antibiotics from the ionophore family of antibiotics is characterized by containing a multiplicity of cyclic ethers in their structures and is generally referred to as a carboxylic polyether ionophore. The antibiotics from the ionophore family of antibiotics are produced by the streptomyces genus of microorganisms. Bergen et al., Journal of Animal Science, 58, pp. 1465-1539; and Kirk-Othmer: Encyclopedia of Chemical Technology, Vol. 3, Third Edition (John Wiley & Sons, Ind., 1978) at page 47.

The antibiotics from the ionophore family of antibiotics may contain more than one factor. Each of the factors may be used in the present invention. It is further anticipated that antibiotics from the ionophore family of antibiotics can form ethers, salts, esters, or other derivatives which are contemplated for use in this invention.

The antibiotics from the ionophore family of antibiotics which can be employed in this invention include Monensin, Ionomycin, Inidlomycin, Nigenicin, Grisorixin, Dianemycin, Lenoremycin, Salinomycin, Narasin, Ionomycin, Arborixin, Septamycin, Etheromycin, Lasalocid (factors A, B, C, D and E), Mutalomycin, Isolasalocid A, and Laidomycin and Lysocellin and factors thereof. A complete list of antibiotics from the ionophore family of antibiotics which can be used in this invention is extensively reviewed and set forth in U.S. Patent 4,405,609. This reference is incorporated herein by reference, and is illustrative of the members of the ionophore family of antibiotics which can be employed in this invention.

Preferred antibiotics from the ionophore family of antibiotics include Monensin, Narasin, Lasalocid, Lysocellin, Salinomycin, Ionomycin, Nigenicin and Dianemycin.

The preferred antibiotic, Monensin, a compound widely used as a growth promotant in animals (see U.S. Patent No. 3,839,557) includes the various active factors A, B, and C, the alkali metal salts thereof such as sodium and the like and the esters thereof such as carbamate esters and the like. Another preferred antibiotic, Narasin, is produced by submerged aerobic fermentation of Streptomyces aureofaciens and includes the active factors A, B and D, esters and physiologically acceptable salts thereof. A typically preferred ester of Narasin includes lower alkyl esters having 1 to 4 carbon atoms such as methyl esters.

An antibiotic from the Virginiamycin family of antibiotics can be administered to animals by any of several alternative methods. They can be orally administered to the animals by forming a homogeneous admixture of an antibiotic from the Virginiamycin family of antibiotics with a commercial dry feed or ration or by initially forming a concentrated premix by mixing the active antibiotic from the Virginiamycin family of antibiotics with a suitable, non-toxic, edible carrier material. Examples of suitable carriers include corn meal, germ meal or other cereals, soy flour, soya grits, seed meal, oyster shell flour, calcium silicate, and the like, and may additionally contain other compatible medicaments.

Generally, the antibiotic from the Virginiamycin family of antibiotics will be administered as a feed additive in the range of about 1 to about 100 grams of active ingredient per ton of feed, preferably from about 5 to about 50 grams of active ingredient per ton of the animal feed.

This invention also provides a composition containing a first component from the Virginiamycin family of antibiotics in combination with a second component from the ionophore family of antibiotics. The components of the present invention are employed in amounts which in combination produce an unexpected synergistic effect.

The combination of the first component from the Virginiamycin family of antibiotics with the second component from the ionophore family of antibiotics can be administered to animals by any of several alternative methods. The compositions of this invention can be orally administered to the animals by forming a homogeneous admixture of the first component from the Virginiamycin family of antibiotics with the second component from the ionophore family of antibiotics with a commercial dry feed or ration. The compositions of this invention can further be orally administered to the animal by initially forming a concentrated premix containing the first and second components of this invention with a suitable, non-toxic, edible carrier material. Examples of such suitable carriers include cornmeal, germ meal or other cereals, soy flour, soya grits, seed meal, oyster shell flour, calcium silicate, and the like, and may contain other compatible medicaments.

The respective components from the Virginiamycin family of antibiotics and the ionophore family of antibiotics will be present on a weight basis in a ratio of about 1 to about 10 parts of the first component from the Virginiamycin family of antibiotics together with about 1 to about 10 parts of the second component from the ionophore family of antibiotics. Generally, the antibiotics from the first and second components will each be administered as a feed additive in the range of about 1 to about 100 grams of active ingredient per ton of feed. Preferably from about 5 to about 50 grams of active ingredient.

A typical feedstuff for feed lot animals has the following composition.

| Ingredient | Percent |
|---|---|
| Coarse ground corn | 69.95 |
| Ground corncobs | 10.00 |
| Soybean meal (50% protein) | 8.00 |
| Alfalfa meal | 5.00 |
| Molasses | 5.00 |
| Urea | 0.60 |
| Dicalcium phosphate | 0.50 |
| Calcium carbonate | 0.50 |
| Salt | 0.30 |
| Vitamin A and $D_2$ premix | 0.07 |
| Vitamin E premix | 0.05 |
| Trace mineral premix | 0.03 |
| | 100.00 |

It is also contemplated that a suitable premix can likewise be prepared by admixing the desired quantity of an antibiotic from the Virginiamycin family of antibiotics or, in the case of the combination, the desired quantity of a first component from the Virginiamycin family of antibiotics and a second component from the ionophore family of antibiotics to a measured amount of commercial feed. If using Virginiamycin alone, premixes in accordance with this invention can advantageously contain from about 0.125% by weight to about 40% by weight of an antibiotic from the Virginiamycin family of antibiotics, preferably from about 0.5 to about 25% by weight, and most preferably about 5% by weight of an antibiotic from the Virginiamycin family of antibiotics. Such premixes may be readily mixed with the feed or ration by conventional techniques in the art. If using the combination, premixes in accordance with this invention can advantageously contain from about 0.125% by weight to about 40% by weight of an antibiotic from the Virginiamycin family of antibiotics, preferably from about 0.5% to about 25% by weight, and most preferably about 1 to about 5% by weight of the Virginiamycin family of antibiotics, in combination with a second component from the ionophore family of antibiotics in an amount of from about 0.25 by weight to about 40% by weight, preferably from about 0.5 to about 25% by weight, and most preferably from about 2 to about 10% by weight of the ionophore family of antibiotics. Such premixes may be readily mixed with the feed or ration by conventional techniques in the art.

It is also contemplated that parenteral therapy may be utilized in this invention as a means of administering the antibiotic from the Virginiamycin family of antibiotics to the animals. A single dosage of from about 10 to about 100 mg., preferably from about 25 to about 50 mg. of the antibiotic from the Virginiamycin family of antibiotics per kilogram of body weight of the animal to be treated is utilized. The dosage regimen for such parenteral therapy may vary according to the age and type of animal, severity of the liver abscesses, and the like. For most therapeutic situations, a single daily dosage is required to be maintained for a period of 4 days. However, parenteral therapy for up to 10 days may be required in the instance of a severe liver abscess. The extent of parenteral dosage may be determined within the discretion of the attending veterinarian.

It is further contemplated that an antibiotic from the Virginiamycin family of antibiotics or the combination of the first component from the Virginiamycin family of antibiotics with the second component from the ionophore family of antibiotics can be administered to the animals in the form of a controlled release formulation such as polymers, waxes and the like. Examples of such slow release excipients include a slow release ruminant pellet, bolus, capsule, implant, or similar prolonged payout device which is capable of effective oral delivery of the first and second components to the animal over a prolonged period of time of up to 3 months or longer.

Controlled release formulations containing a composition of this invention and a carrier can be prepared by methods known in the art. See, for example, U.S. Patent Nos. 4,333,919; 4,331,652; and 4,293,539.

For example, the controlled release formulations can be prepared by mixing an antibiotic from the Virginiamycin family of antibiotics or the combination consisting of a first component from the Virginiamycin family of antibiotics with a second component from the ionophore family of antibiotics with a copolymer derived from the condensation of about 60 to about 95 parts by weight of lactic acid and about 5 to about 40 parts by weight glycolic acid. The mixture so formed can be molded into a tablet, or placed inside of a bolus for oral administration to the animal. The formulated composition is slowly degraded so that the animal will receive an effective amount of the first and second component over an extended period of time. This method is particularly effective in ruminant containing animals.

It is also contemplated that a parenteral therapy may be utilized in this invention as a means of administering the combination of the first component from the Virginiamycin family of antibiotics with the second component from the ionophore family of antibiotics to the animals. A single dosage of from about 0.2 to about 2 mg., preferably from about 0.5 mg. to about 1 mg. of a first component from the Virginiamycin family of antibiotics and the second component from the ionophore family of antibiotics of about 0.3 to about 3 mg., preferably 1 mg. per kilogram of body weight is contemplated.

It is preferable to orally administer the antibiotic from the Virginiamycin family of antibiotics via the animal's normal feed ration.

In this invention, an effective but non-toxic quantity of the antibiotic from the Virginiamycin family of antibiotics or the combination of an antibiotic from the Virginiamycin family of antibiotics with an antibiotic from the ionophore family of antibiotics are administered to monogastric or ruminant, meat-or milk-producing animals for use in treating liver abscesses. The animals which can be treated by this invention include beef cattle, dairy cattle, sheep, swine, poultry, horses and rabbits. The preferred animals which can be treated by this invention include beef cattle and dairy cattle.

## EXAMPLES

Examples provided are intended to assist in a further understanding of this invention. Particular materials employed, species and conditions, are intended to be further illustrative of the invention and not limitative of the reasonable scope thereof.

## EXAMPLE I

A cattle ration was prepared using the following formula:

|  | Proportion (%) | |
| --- | --- | --- |
|  | As Is | Air Dry |
| Corn | 69.0 | 82.0 |
| Alfalfa hay | 2.5 | 3.0 |
| Corn Silage | 24.5 | 10.0 |
| Supplement | 4.0 | 5.0 |

The ration was supplemented with varying proportions of Virginiamycin and fed, ad libitum, to approximately 200 cattle weighing between 650 lbs. and 850 lbs. The cattle were processed according to standard procedures for the facility, individually weighed and identified. The treatments were continued until the cattle reached an approximate weight of 1100 lbs. (approximately 120 days).

Virginiamycin was added to the ration in the following amounts:

Table 2

| Drug | Virginiamycin Target Dose mg/hd/da | Approx. Inclusion Rate Based on Dry Ration |
| --- | --- | --- |
| Control | 0 | 0 |
| Vm | 100 | 9.1g/Ton |
| Vm | 250 | 22.7g/Ton |
| Vm | 500 | 45.5g/Ton |

Vm = Virginiamycin

At slaughter, livers were identified with the individual animal number and scored for liver abscess after examination in the manner required by USDA. Livers were palpated and suspicious area were excised and examined for the presence of liver abscesses.

The incidence of liver abscess is summarized below:

Table 3

Liver Abscess

| (grams/ton) | Vm | | | |
|---|---|---|---|---|
| | 0 | 9.1 | 22.7 | 45.5 |
| No. of Animals With Liver Abscess | 26 | 16 | 12 | 6 |
| No. of Animals Without Liver Abscess | 173 | 181 | 185 | 191 |
| Total Animals | 199 | 197 | 197 | 197 |

There was an obvious beneficial effect of Virginiamycin on the incidence of liver abscess. This data was analyzed by a Chi-Square Goodness of Fit Procedure. There was a measurable difference among the groups ($x^2$-15.01, P=0018).

## EXAMPLE 2

In this example, microbiological studies were initiated with Virginiamycin and other antibiotics so as to determine their potency in controlling liver abscesses. As Fusobacterium necrophorum has been associated with bovine liver abscesses in cattle, it was determined that microbiological studies involving this bacterium would be undertaken with representative examples of antibiotics including Virginiamycin.

The MIC's for Fusobacterium necrophorum was done by the agar dilution method. Two-fold serial dilutions of each compound was incorporated into Wilkins-Chalgren Agar plates. Each Fusobacterium necrophorum was propagated in Peptone-Yeast-Glucose broth and inoculated onto the plates with a multipoint inoculating device. The plates were
incubated at 37°C in an atmosphere of 10% $H_2$, 5% $CO_2$ and 85% $N_2$, for 40 hours.

## Table I

Minimal Inhibitory Concentrations (MICs) of Feed
Additives Containing Various Antibiotics Against
Three Strains of Fusobacterium necrophorum (ATCC
25286, ATCC 27852 and the Colorado Strain)

| | MIC, μg/ml | | |
|---|---|---|---|
| Feed additive | ATCC 25286 | ATCC 27852 | Colorado Strain |
| Virginiamycin | 0.9* | 1.0* | 1.0* |
| Factor M | 0.5 | 0.25 | |
| Factor S | 32.0 | 32.0 | |
| Tylosin | 0.8* | 1.4* | 1.0* |
| Erythromycin | 8.0 | | |
| Monensin | 6.4* | 3.0 | 4.2* |
| Salinomycin | 3.6* | 4.0 | 5.7* |
| AB102-5 | 1.0* | | |
| Lasalocid | 9.1* | 12.0 | 8.5* |
| Oxytetracyline | 0.03 | 0.25 | |
| Chlortetracycline | 0.02 | 0.12 | |
| Flavomycin | 0.25 | 0.12 | |
| Zinc bacitracin | | 9.2 | |
| AAD216 | 67.0* | 39.0* | 48.0* |
| Avoparcin | 32.0 | 64.0 | |

*Geometric mean of more than one determination from
different experiments.

Table I indicates that Virginiamycin was twice as effective as Tylosin against the strain of Fusobacterium necrophorum ATCC 27852, and was comparable to Tylosin against the other two strains. Both were less effective (higher MICs) than Chlortetracycline, Oxytetracycline and Flavomycin. Virginiamycin was further superior (lower MICs) than Erythromycin, the ionophores, zinc Bacitracin and the glycopeptides AAD216 and Avoparcin. In the one experiment in which factors M and S of virginiamycin were tested, the MICs were 0.35 (geometric mean for the two ATCC strains) and greater than 32 μg/ml, respectively. Virginiamycin contains 75% of Factor M and 25% of Factor S.

EXAMPLE 3

In this example, microbiological studies were initiated with combinations of antibiotics from the Virginiamycin family of antibiotics and ionophore family of antibiotics so as to determine their potency in controlling liver abscesses. As Fusobacterium necrophorum has been associated with bovine liver abscess in cattle, it was determined that microbiological studies involving this bacterium would be undertaken. Since the liver abscess etiology is poorly defined, it is difficult to precisely extrapolate these studies to animal disease. However, the responses set forth in the table below clearly suggest that combinations of antibiotics from the virginiamycin family of antibiotics and the ionophore family of antibiotics would be useful in controlling the primary pathogen associated with liver abscesses.

Table II

Effects of Virginiamycin on Fusobacterium
necrophorum when Combined with Ionophores

| | | MIC (µg/ml) | |
|---|---|---|---|
| | Ratio | Alone | Combina-tion |
| Ferrensin-mycin | 1.3 | 0.5 | 0.6 |
| Virginiamycin | 1 | 1 | 0.5 |
| Fasalocid | 2 | 7 | 1 |
| Virginiamycin | 1 | 0.7 | 1 |
| Fasalocid | 3 | 10 | 3 |
| Virginiamycin | 1 | 1 | 0.9 |
| Monensin | 0.7 | 4 | 0.4 |
| Virginiamycin | 1 | 1 | 0.5 |
| Monensin | 2 | 4 | 0.02 |
| Virginiamycin | 1 | 1 | 0.01 |
| Monensin | 3 | 4 | 2 |
| Virginiamycin | 1 | 1 | 0.7 |
| Salinomycin | 1 | 5 | 0.7 |
| Virginiamycin | 1 | 1 | 0.7 |
| Salinomycin | 1.3 | 5 | 0.02 |
| Virginiamycin | 1 | 1 | 0.02 |

Virginiamycin was combined with an antibiotic from the ionophore family in a ratio expected in medicated feeds, eg. Salinomycin at 20 g/Ton and Virginamycin at 15 g/Ton have a ratio of 1.3:1. For the microbilogical studies, a stock solution was made up to contain Salinomycin and Virginiamycin in the same ratio of 1.3:1. The stock solution was serially diluted two fold. The dilutions were incubated with F. necr. The end point was the lowest concentrations antibiotics which inhibited the growth of F. necr., or MIC for minimal inhibitory concentration, eg. the MIC of Salinomycin was 5 µg/ml while that of Virginiamycin was 1 µg/ml.

Synergism was noted when there was a four fold, or greater, decrease in the MIC of either antibiotic, eg. combined at the 1.3:1 ratio, the MIC of Salinomycin was decreased 250-fold to 0.02 µg/ml while that of Virginiamycin 50-fold to 0.02 µg/ml.

In Table II, besides the synergistic Salinomycin-Virginiamycin combinations both Fasalocid-Virginiamycin and Monensin-Virginiamycin at the 2:1 ratio exhibited synergism. However, the magnitude of synergism of Monensin-Virginiamycin was reduced 20-fold when the ratio was changed to 0.7:1 while the effect of synergism became non existent when the ratio was changed to 3:1.

## Claims

1. An animal feed composition comprising a first component from the Virginiamycin family of antibiotics and a second component from the ionophore family of antibiotics.

2. The composition of claim 1, wherein the first component from the Virginiamycin family of antibiotics is Madumycin, Mikamycin, Ostreogrycin, Patricin, Plauracin, Pristinamycin, Streptogramin, Synergistins, Vernamycin or Virginiamycin or factors thereof.

EP 0 356 192 A2

3. The composition of claim 1 or claim 2, wherein the second component from the ionophore family of antibiotics is Monensin, Ionomycin, Inidlomycin, Nigenicin, Grisorixin, Dianemycin, Lenoremycin, Salinomycin, Narasin, Ionomycin, Alborixin, Septamycin, Etheromycin, Lasalocid, Mutalomycin, Isolasalocid A, Laidomycin or Lysocellin, or factors thereof.

4. The composition of any one of claims 1 to 3, wherein the first component from the Virginiamycin family of antibiotics is Virginiamycin.

5. The composition of claim 4, wherein the first component from the Virginiamycin family of antibiotics is Factor M of Virginiamycin.

6. The composition of any one of claims 1 to 5, wherein the second component from the ionophore family of antibiotics is Monensin, Narasin, Lasalocid, Salinomycin, Lonomycin, Nigenicin or Dianemycin.

7. The composition of claim 6 wherein the second component from the ionophore family of antibiotics is Monensin or Lasalocid.

8. The composition of any one of claims 1 to 7, wherein the amount of the first component from the Virginiamycin family of antibiotics ranges from about 1 to about 100 grams of antibiotic per ton of feed; and the amount of the second component from the ionophore family of antibiotics ranges from about 1 to about 100 grams per ton of feed.

9. The composition of claim 8, wherein the amount of the first component from the Virginiamycin family of antibiotics ranges from about 5 to about 50 grams of antibiotic per ton of feed; and the amount of the second component from the ionophore family of antibiotics ranges from about 5 to about 50 grams per ton of feed.

10. The composition of any one of claims 1 to 9, wherein the composition is a whole feed ration.

11. The composition of any one of claims 1 to 9, wherein the composition is in the form of a release formulation.

12. The composition of any one of claims 1 to 9, wherein the composition is in the form of a premix.

13. The use of an antibiotic from the Virginiamycin family of antibiotics in the manufacture of a medicament for treating and preventing liver abscesses in animals.

14. The use according to claim 13 in which the antibiotic from the Virginiamycin family of antibiotics is Madumycin, Mikamycin, Ostreogrycin, Patricin, Plauracin, Pristinamycin, Streptogramin, Synergistins, Vernamycin or Virginiamycin, or factors thereof.

15. The use according to claim 14 in which the antibiotic from the Virginiamycin family of antibiotics is Virginiamycin.

16. The use according to claim 15 in antibiotic from the Virginiamycin family is Factor M of Virginiamycin.

**Claims for the following Contracting States: ES, GR.**

1. A process for preparing an animal feed composition which comprises bringing into association a first component from the Virginiamycin family of antibiotics and a second component from the ionophore family of antibiotics.

2. A process for preparing the composition of claim 1, wherein the first component from the Virginiamycin family of antibiotics is Madumycin, Mikamycin, Ostreogrycin, Patricin, Plauracin, Pristinamycin, Streptogramin, Synergistins, Vernamycin or Virginiamycin or factors thereof.

3. A process for preparing the composition of claim 1 or claim 2, wherein the second component from the ionophore family of antibiotics is Monensin, Ionomycin, Inidlomycin, Nigenicin, Grisorixin, Dianemycin, Lenoremycin, Salinomycin, Narasin, Ionomycin, Alborixin, Septamycin, Etheromycin, Lasalocid, Mutalomycin, Isolasalocid A, Laidomycin or Lysocellin, or factors thereof.

4. A process for preparing the composition of any one of claims 1 to 3, wherein the first component from the Virginiamycin family of antibiotics is Virginiamycin.

5. A process for preparing the composition of claim 4, wherein the first component from the Virginiamycin family of antibiotics is Factor M of Virginiamycin.

6. A process for preparing the composition of any one of claims 1 to 5, wherein the second component from the ionophore family of antibiotics is Monensin, Narasin, Lasalocid, Salinomycin, Lonomycin, Nigenicin or Dianemycin.

7. A process for preparing the composition of claim 6 wherein the second component from the ionophore family of antibiotics is Monensin or Lasalocid.

8. A process for preparing the composition of any one of claims 1 to 7, wherein the amount of the first component from the Virginiamycin family of antibiotics ranges from about 1 to about 100 grams of antibiotic per ton of feed; and the amount of the second component from the ionophore family of antibiotics ranges from about 1 to about 100 grams per ton of feed.

9. A process for preparing the composition of claim 8, wherein the amount of the first component from the Virginiamycin family of antibiotics ranges from about 5 to about 50 grams of antibiotic per ton of feed; and the amount of the second component from the ionophore family of antibiotics ranges from about 5 to about 50 grams per ton of feed.

10. A process for preparing the composition of any one of claims 1 to 9, wherein the composition is a whole feed ration.

11. A process for preparing the composition of any one of claims 1 to 9, wherein the composition is in the form of a controlled release formulation.

12. A process for preparing the composition of any one of claims 1 to 9, wherein the composition is in the form of a premix.

13. The use of an antibiotic from the Virginiamycin family of antibiotics in the manufacture of a medicament for treating and preventing liver abscesses in animals.

14. The use according to claim 13 in which the antibiotic from the Virginiamycin family of antibiotics is Madumycin, Mikamycin, Ostreogrycin, Patricin, Plauracin, Pristinamycin, Streptogramin, Synergistins, Vernamycin or Virginiamycin, or factors thereof.

15. The use according to claim 14 in which the antibiotic from the Virginiamycin family of antibiotics is Virginiamycin.

16 The use according to claim 15 in which the antibiotic from the Virginiamycin family is Factor M of Virginiamycin.

11